# EUROPÄISCHE PATENTSCHRIFT

(11) **EP 0 559 814 B1**
(45) Veröffentlichungstag und Bekanntmachung des Hinweises auf die Patenterteilung: **09.09.1998**
(21) Anmeldenummer: 92902574.0
(22) Anmeldetag: 21.11.1991
(51) Int. Cl.: C07D 521/00, C07D 251/16, A01N 47/36

(54) **HERBIZIDE N- (1,3,5-TRIAZIN-2-YL)AMINOCARBONYL]BENZOLSULFONAMIDE**
HERBICIDAL N- (1,3,5-TRIAZINE-2-YL)AMINOCARBONYL]BENZOLE SULPHONAMIDES
N- (1,3,5-TRIAZIN-2-YL)AMINOCARBONYL]BENZOLSULFONAMIDES HERBICIDES

(30) Priorität: 01.12.1990 DE 4038430
(43) Veröffentlichungstag der Anmeldung: 15.09.1993
(73) Patentinhaber: BASF Aktiengesellschaft, 67063 Ludwigshafen (DE)
(72) Erfinder: MAYER, Horst, D-6700 Ludwigshafen (DE); HAMPRECHT, Gerhard, D-6940 Weinheim (DE); WESTPHALEN, Karl-Otto, D-6720 Speyer (DE); WALTER, Helmut, D-6719 Obrigheim (DE); GERBER, Matthias, D-6704 Mutterstadt (DE); GROSSMANN, Klaus, D-6703 Limburgerhof (DE); RADEMACHER, Wilhelm, D-6703 Limburgerhof (DE)
(86) Internationale Anmeldenummer: EP9102192
(87) Internationale Veröffentlichungsnummer: WO9209608

(56) Entgegenhaltungen:
- EP-A- 44 807
- EP-A- 44 808
- EP-A- 48 143
- EP-A- 388 873
- US-A- 4 127 405
- US-A- 4 169 719

## Beschreibung

Die vorliegende Erfindung betrifft N-[(1,3,5-Triazin-2-yl)-aminocarbonyl]benzolsulfonamide der allgemeinen Formel I 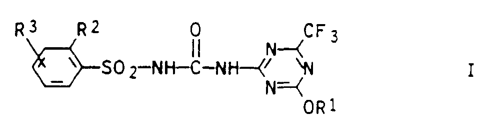 in der R¹ eine Methyl- oder Ethylgruppe, R² Halogen, eine C₁-C₃-Alkylsulfonylgruppe, die Trifluormethylgruppe oder die 2-Methoxyethoxygruppe, und R³ Wasserstoff, die Methyl-, Ethyl-, Methoxy- oder Ethoxygruppe, Fluor oder Chlor bedeuten, sowie deren landwirtschaftlich brauchbare Salze.

Des weiteren betrifft die Erfindung ein Verfahren zur Herstellung der Verbindungen I sowie ihre Verwendung als herbizide Mittel.

Das US-Patent 4 127 405 und die EP-A 44 807 betreffen Sulfonylharnstoffe mit herbizider Wirkung deren allgemeine Formel die eingangs definierten Verbindungen der allgemeinen Formel I umfaßt.

In der US 4 127 405 ist als nächtliegende Struktur die Triazinverbindung A sowie in der US 4 169 719 das Pyrimidin-Derivat B beschrieben.

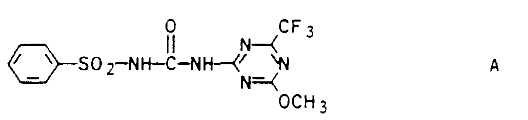

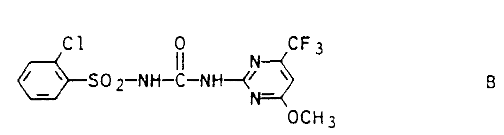

In der EP-A 44 807 sind zwei Sulfonylharnstoffe C mit einer ortho-ständigen Allyloxygruppe beschrieben. 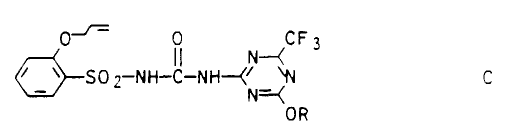 R=CH₃, C₂H₅

In der EP-A 44 808 sind zwei Sulfonylharnstoffe D mit einer 2-Chlorethoxysubstitution im Aromatenteil beschrieben. 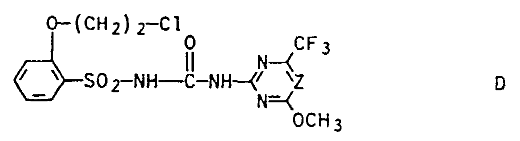 Z = CH,N

In der EP-A 48 143 sind zwei N-methylierte Sulfonylharnstoffe E ohne nähere Charakterisierung aufgeführt. 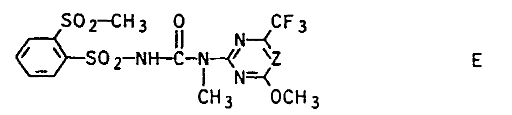 Z = CH,N

Die EP-A 388 873 umfaßt Benzoesäureester der Struktur F. 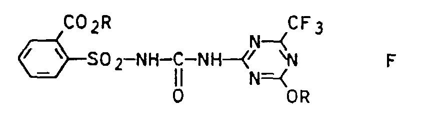 R = CH₃,C₂H₅

Das US-Patent 4 127 405 offenbart Sulfonylharnstoffderivate mit Chlor- bzw. Trifluormethylsubstitution in ortho-Stellung des Phenylrings und CH₃/OCH₃-Substitution im Triazinring 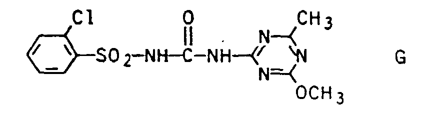 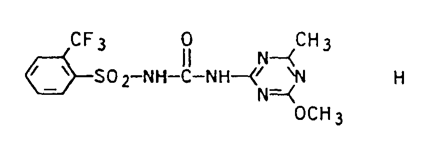 Die Verbindung G ist unter der Bezeichnung Chlorsulfuron (Glean®) bekannt.

Der Erfindung lag nun die Aufgabe zugrunde, Sulfonylharnstoffe zu synthetisieren, die gegenüber den bekannten Vertretern dieser Herbizid-Klasse verbesserte Eigenschaften aufweisen und sich insbesondere durch hohe Selektivität in empfindlichen Kulturen wie Reis oder Mais auszeichnen.

Entsprechend dieser Aufgabe wurden die eingangs definierten N-[(1,3,5-Triazin-2-yl)aminocarbonyl]benzolsulfonamide der Formel I gefunden.

In der Formel I bedeutet C₁-C₃-Alkylsulfonyl eine Methyl-, Ethyl-, Propyl- oder Isopropylsulfonylgruppe, Halogen steht für Fluor, Chlor, Brom oder Jod, insbesondere Fluor oder Chlor. Besonders bevorzugt sind Benzolsulfonamide, in den R² für Chlor steht.

Die erfindungsgemäßen Sulfonylharnstoffe der Formel I sind auf verschiedenen Wegen zugänglich, die in der Literatur beschrieben sind. Beispielhaft seien besonders vorteilhafte Wege (A-D) im folgenden näher erläutert. 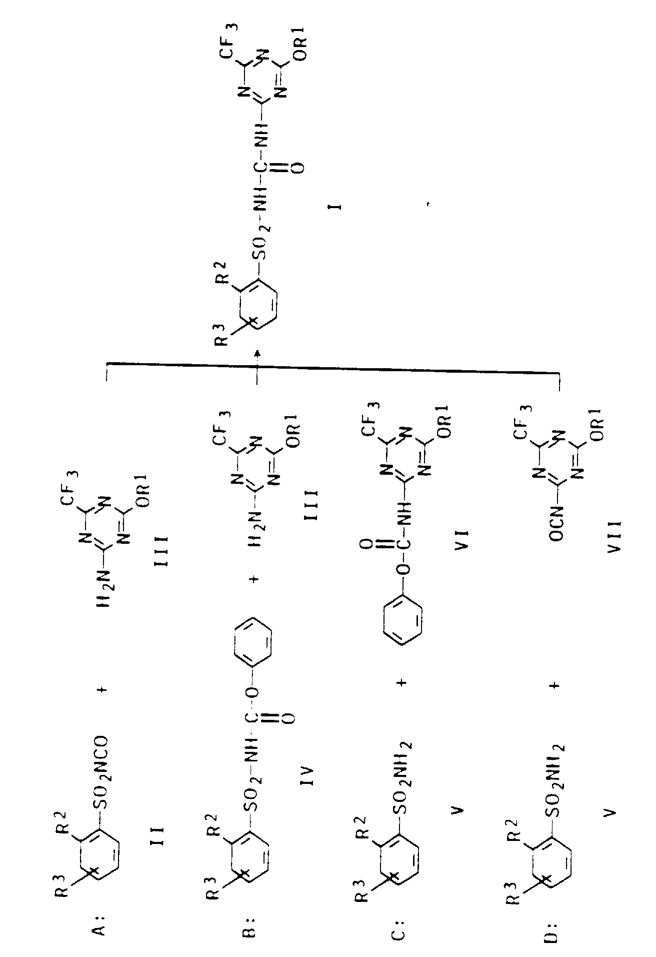
A: Man setzt ein Sulfonylisocyanat II in an sich bekannter Weise (EP-A-162 723) mit ungefähr der stöchiometrischen Menge eines 2-Amino-1,3,5-triazin-derivats III bei einer Temperatur von 0 bis 120°C, vorzugsweise 10 bis 100°C um. Die Reaktion kann unter Normaldruck oder unter Druck (bis 50 bar), vorzugsweise bei 1 bis 5 bar, kontinuierlich oder diskontinuierlich durchgeführt werden.
   Zweckmäpigerweise verwendet man für die Umsetzungen unter den jeweiligen Reaktionsbedingungen inerte Lösungs- und Verdünnungsmittel. Als Lösungsmittel kommen beispielsweise Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan,
   o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, β,β-Dichlordiethyl-ether;
   Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester, z.B. Ethylacetat, Acetessigester, Isobutylacetat; Amide, z.B. Formamid, Methylformamid, Dimethylformamid; Ketone, z.B. Aceton, Methylethylketon und entsprechende Gemische in Betracht. Zweckmäßig verwendet man das Lösungsmittel in einer Menge von 100 bis 2000 Gew.%, vorzugsweise von 200 bis 700 Gew.%, bezogen auf den Ausgangsstoff II.
   Die zur Umsetzung benötigte Verbindung II wird im allgemeinen in etwa äquimolaren Mengen (mit einem über- oder Unterschuß von z.B. 0 bis 20 %, bezogen auf den jeweiligen Ausgangsstoff III) eingesetzt. Man kann den Ausgangsstoff III in einem der vorgenannten Verdünnungsmittel vorlegen und dann den Ausgangsstoff II zugeben.
   Zweckmäßigerweise wird das Verfahren zur Herstellung der neuen Verbindungen jedoch so durchgeführt, daß man den Ausgangsstoff II, gegebenenfalls in einem der vorgenannten Verdünnungsmittel, vorlegt und dann den Ausgangsstoff III zugibt.
   Zur Beendigung der Umsetzung rührt man nach der Zugabe der Komponenten noch 20 Minuten bis 24 Stunden bei 0 bis 120°C, vorzugsweise 10 bis 100°C, nach.
   Als Reaktionsbeschleuniger kann man vorteilhafterweise ein tertiäres Amin, z.B. Pyridin, α,β,γ-Picolin, 2,4-, 2,6-Lutidin, 2,4,6-Collidin, p-Dimethylaminopyridin, Trimethylamin, Triethylamin, Tri(n-propyl)amin, 1,4-Diaza[2,2,2]bi-cyclooctan [DABCO] oder 1,8-Diazabicylcol[5,4,0]-undec-7-en in einer Menge von 0.01 bis 1 Mol pro Mol Ausgangsstoff II verwenden.
   Aus dem Reaktionsgemisch wird der Endstoff I in üblicher Weise, z.B. nach Abdestillieren von Lösungsmittel oder direkt durch Absaugen isoliert. Der verbleibende Rückstand kann noch mit Wasser bzw. verdünnter Säure zur Entfernung basischer Verunreinigungen gewaschen werden. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Rühren in einem organischen Lösungsmittel, das die Verunreinigungen aufnimmt oder Chromatographie gereinigt werden.
   Bevorzugt führt man diese Umsetzung in Acetonitril, Methyl-tert.-butylether, Toluol oder Methylenchlorid in Anwesenheit von 0 bis 100 Moläquivalenten, vorzugsweise 0 bis 50 Moläquivalenten eines tertiären Amins wie 1,4-Diazabicyclo[2,2,2]octan oder Triethylamin durch.
B: Man setzt ein entsprechendes Sulfonylcarbamat der Formel IV in an sich bekannter Weise (EP-A-120 814, EP-A-101 407) in einem inerten organischen Lösungsmittel bei einer Temperatur zwischen 0 und 120°C, vorzugsweise 10 bis 100°C mit einem 2-Amino-1,3,5-triazin-derivat III um. Es können hierbei Basen wie tertiäre Amine zugesetzt werden, wodurch die Reaktion beschleunigt und die Produktqualität verbessert wird.
   Geeignete Basen hierfür sind z.B. tertiäre Amine wie unter A angegeben, insbesondere Triethylamin oder 1,4-Diazabicyclo[2,2,2]octan, in einer Menge von 0,01 bis 1 mol pro Mol Ausgangsstoff IV.
   Zweckmäßig verwendet man als Lösungsmittel die unter A angegebenen.
   Man verwendet das Lösungsmittel in einer Menge von 100 bis 2.000 Gew.%, vorzugsweise von 200 bis 700 Gew.%, bezogen auf den Ausgangsstoff IV.
   Die zur Umsetzung benötigte Verbindung IV wird im allgemeinen in etwa äquimolaren Mengen (mit einem über- oder Unterschuß von z.B. 0 bis 20 %, bezogen auf jeweiligen Ausgangsstoff III eingesetzt. Man kann den Ausgangsstoff IV in einem der vorgenannten Verdünnungsmittel vorlegen und dann den Ausgangsstoff III zugeben.
   Man kann jedoch auch den Ausgangsstoff III in einem der genannten Löse- oder Verdünnungsmittel vorlegen und das Sulfonylcarbamat IV zugeben.
   In beiden Fällen kann als Katalysator vor oder während der Reaktion eine Base zugesetzt werden.
   Aus dem Reaktionsgemisch kann das Endprodukt I in üblicher Weise, wie unter A angegeben, gewonnen werden.
C: Man setzt ein Sulfonamid der Formel V in an sich bekannter Weise (EP-A-141 777 und EP-A-101 670) in einem inerten organischen Lösungsmittel mit ungefähr der stöchiometrischen Menge eines Phenylcarbamats VI bei einer Temperatur von 0 bis 120°C, vorzugsweise 20 bis 100°C um. Die Reaktion kann bei Normaldruck oder unter Druck (bis 50 bar), vorzugsweise bei 1 bis 5 bar, kontinuierlich oder diskontinuierlich durchgeführt werden.
   Es können hierbei Basen wie tertiäre Amine zugesetzt werden, die die Reaktion beschleunigen und die Produktqualität verbessern. Geeignete Basen sind hierfür die unter A angegebenen, insbesondere Triethylamin, 2,4,6-Collidin, 1,4-Diazabicyclo[2,2,2]octan [DABCO] oder 1,8-Diazabicyclo-[5,4,0]undec-7-en (DBU), in einer Menge von 0,01 bis 1 mol pro Mol Ausgangsstoff V.
   Zweckmäßigerweise verwendet man als Löse- oder Verdünnungsmittel die unter A angegebenen.
   Man verwendet das Lösungsmittel in einer Menge von 100 bis 2000 Gew.%, vorzugsweise von 200 bis 700 Gew.%, bezogen auf das Edukt V.
   Die zur Umsetzung benötigte Verbindung V wird im allgemeinen in etwa äquimolaren Mengen (mit einem über- oder Unterschuß von z.B. 0 bis 20 %, bezogen auf die jeweiligen Ausgangsstoffe VI) eingesetzt. Man kann den Ausgangsstoff VI in einem der vorgenannten Verdünnungsmittel vorlegen und dann den Ausgangsstoff V zugeben.
   Man kann jedoch auch den Ausgangsstoff V in einem der genannten Lösungsmittel vorlegen und dann das Carbamat VI zugeben. In beiden Fällen kann als Katalysator vor oder während der Reaktion eine der genannten Basen zugesetzt werden.
   Zur Beendigung der umsetzung rührt man nach der Zugabe der Komponenten noch 20 Minuten bis 24 Stunden bei 0 bis 120°C, vorzugsweise 10 bis 100°C, insbesondere 20 bis 80°C, nach.
   Man isoliert die Sulfonylharnstoffe der Formel I aus dem Reaktionsgemisch mit den üblichen Methoden, wie unter A beschrieben.
D: Man setzt ein Sulfonamid der Formel V in an sich bekannter Weise (EP-A-234 352) in einem inerten organischen Lösungsmittel mit ungefähr der stöchiometrischen Menge eines Isocyanates VII zu einer Temperatur von 0 bis 150°C, vorzugsweise 10 bis 100°C um. Die Reaktion kann bei Normaldruck oder unter Druck (bis 50 bar), vorzugsweise bei 1 bis 5 bar, kontinuierlich oder diskontinuierlich durchgeführt werden.
   Es können hierbei vor oder während der Reaktion Basen wie tertiäre Amine zugesetzt werden, die die Reaktion beschleunigen und die Produktqualität verbessern. Geeignete Basen sind hierfür die unter A angegebenen, insbesondere Triethylamin oder 2,4,6-Collidin, in einer Menge von 0,01 bis 1 mol pro Mol Ausgangsstoff V.
   Zweckmäßigerweise verwendet man als Lösungsmittel die unter A angegebenen. Man setzt das Lösungsmittel in einer Menge von 100 bis 2000 Gew.%, vorzugsweise von 200 bis 700 Gew.%, bezogen auf das Edukt V.
   Die zur Umsetzung benötigte Verbindung V wird im allgemeinen in etwa äquimolaren Mengen (mit einem über- oder Unterschuß von z.B. 0 bis 20 %, bezogen auf die Edukte VII) eingesetzt. Man kann den Ausgangsstoff VII in einem der genannten Verdünnungsmittel vorlegen und dann den Ausgangsstoff V zugeben. Man kann aber auch das Sulfonamid vorlegen und dann das Isocyanat VII zugeben.
   Zur Beendigung der Umsetzung rührt man nach der Zugabe der Komponenten noch 20 Minuten bis 24 Stunden bei 0 bis 120°C, vorzugsweise 10 bis 100°C, insbesondere 20 bis 80°C, nach. Das Endprodukt I kann aus dem Reaktionsgemisch in der üblichen Weise, wie unter A: beschrieben, gewonnen werden.

Die als Ausgangsstoffe benötigten Sulfonylisocyanate der Formel II lassen sich in an sich bekannter Weise aus den entsprechenden Sulfonamiden durch Phosgenierung (Houben-Weyl 11/2 (1985) 1106, US 4 379 769) oder durch Umsetzung der Sulfonamide mit Chlorsulfonylisocyanat (DE-OS 3 132 944) gewinnen.

Die Sulfonylcarbamate der Formel IV wurden nach oder in Analogie zu an sich bekannten Reaktionen (z.B. EP-A 120 814) hergestellt. Man kann aber auch die Sulfonylisocyanate der Formel II in glatter Reaktion in einem inerten Lösungsmittel wie Ether oder Dichlormethan mit Phenol in die Carbamate der Formel IV überführen.

Carbamate der Formel VI sind nach oder in Analogie zu bekannten Umsetzungen (z.B. EP-A 101 670) zugänglich, sie lassen sich aber auch aus den entsprechenden Isocyanaten VII durch Umsetzung mit Phenol herstellen.

Die Isocyanate der Formel VII erhält man aus den Aminen der Formel III durch Behandlung mit Oxalylchlorid oder Phosgen (in Analogie nach Angew. Chem. 83 (1971) 407, EP-A 388 873).

Die Sulfonamide lassen sich durch Reaktion der entsprechenden Sulfonsäurechloride mit Ammoniak gewinnen (Houben-Weyl, Methoden der organischen Chemie, Band 9 (1955) 605). Die Sulfonsäurechloride erhält man entweder durch Meerwein-Reaktion (Diazotierung geeigneter Amine und Kupfersalz-katalysierte Sulfochlorierung) oder durch Chlorsulfonierung geeigneter Aromaten beispielsweise 2,5-Dichlorbenzolsulfonsäurechlorid aus p-Dichlorbenzol (Houben-Weyl, Methoden der organischen Chemie, Band 9 (1955) 557 ff.).

2-Amino-4-methoxy-6-trifluormethyl-1,3,5-triazin und 2-Amino-4-ethoxy-6-trifluormethyl-1,3,5-triazin sind literaturbekannt (Yakugaku Zasshi 95 (1975) 499).

Die Salze der Verbindungen I sind in an sich bekannter Weise zugänglich (EP-A-304 282, US-A 4,599,412). Man erhält sie durch Deprotonierung der entsprechenden Sulfonylharnstoffe I in Wasser oder einem inerten organischen Lösungsmittel bei Temperaturen von -80°C bis 120°C, vorzugsweise 0°C bis 60°C in Gegenwart einer Base.

Geeignete Basen sind beispielsweise Alkali- oder Erdalkalimetallhydroxide, -hydride, -oxide oder -alkoholate wie Natrium-, Kalium- und Lithiumhydroxid, Natriummethanolat, -ethanolat und -tert.-butanolat, Natrium- und Calziumhydrid und Calziumoxid.

Als Lösungsmittel kommen beispielsweise neben Wasser auch Alkohole wie Methanol, Ethanol und tert.-Butanol, Ether wie Tetrahydrofuran und Dioxan, Acetonitril, Dimethylformamid, Ketone wie Aceton und Methylethylketon und auch halogenierte Kohlenwasserstoffe in Betracht.

Die Deprotonierung kann bei Normaldruck oder bei Drucken bis 50 bar, vorzugsweise bei Normaldruck bis 5 bar Überdruck durchgeführt werden.

Die Verbindungen I bzw. die sie enthaltenden herbiziden Mittel sowie deren umweltverträgliche Salze von Alkalimetallen und Erdalkalimetallen können in Kulturen wie Weizen, Reis und Mais Schadpflanzen sehr gut bekämpfen, ohne die Kulturpflanzen zu schädigen, ein Effekt, der vor allem auch bei niedrigen Aufwandmengen auftritt. Sie können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder öldispersionen. Als inerte Zusatzstoffe kommen u.a. Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier-oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether-und Fettalkoholsulfaten, sowie Salze sulfatierter Hexa-, Hepta-und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkyl-ether oder Polyoxypropylen alkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff.

Beispiele für Formulierungen sind:
I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.
II. 20 Gewichtsteile der Verbindung Nr. 1 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
III. 20 Gewichtsteile der Verbindung Nr. 1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
V. 20 Gewichtsteile des Wirkstoffs Nr. 1 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
VI. 3 Gewichtsteile des Wirkstoffs Nr. 1 werden mit 97 Gewichtsteilen feinteiligem Kaolin vermischt. Man erhält auf diese Weise ein Stäubemittel, das 3 Gew.% des Wirkstoffs enthält.
VII. 30 Gewichtsteile des Wirkstoffs Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gewichtsteile des Wirkstoffs Nr. 1 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 1,0 kg/ha, vorzugsweise 0,01 bis 0,5 kg/ha.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor - Färberdistel |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanan |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Solanum tuberosum | Kartoffel |
| Sorghum bicolor (S. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die Triazinyl-substituierten Sulfonylharnstoffe der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Phenyloxy- bzw. Heteroaryloxy-phenylpropionsäuren sowie deren Salze, Ester und Amide und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Nachstehend sind Beispiele für die Synthese der Verbindungen I wiedergegeben.
1) 2-Chlor-1-N-[4-methoxy-6-trifluormethyl-1,3,5-triazin-2-yl)a minocarbonyl]benzolsufonamid 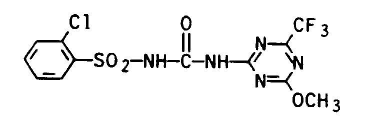 Eine Lösung aus 4,0 g (21 mmol) 2-Amino-4-methoxy-6-trifluormethyl-1,3,5-triazin in 20 ml Acetonitril wurde bei 25°C mit 4,5 g (21 mmol) 2-Chlorbenzolsulfonylisocyanat versetzt. Die so erhaltene Lösung wurde 21 Stunden bei 25°C gerührt. Das Lösungsmittel wurde dann unter vermindertem Druck bei 40°C entfernt und der feste Rückstand mit 1 l eines Diethylether-/Hexan-Gemisches (v:v 1/1) 3 h kräftig gerührt. Das Produkt wurde abgesaugt und im Vakuum bei 40°C getrocknet. Man erhielt 6,5 g (75 % d.Th.) der Titelverbindung mit Fp. 166 - 168°C.
2) Natrium[2-chlor-1-N-[(4-methoxy-6-trifluormethyl-1,3,5-triazin-2-yl)aminocarbonyl)benzolsulfonamid]
   Eine Suspension von 1,5 g (3,6 mmol) 2-Chlor-1-[(4-methoxy-6-trifluormethyl-1,3-5-triazin-2-yl)aminocarbony]benzolsulfonamid in 10 ml Methylenchlorid wurde bei 25°C mit 0,66 g (3,6 mmol) einer Lösung von Natriummethanolat (30 gew.-prozentig) in Methanol versetzt. Die sich bildende homogene Lösung wurde 1 h bei 25°C gerührt. Nach Entfernen der flüchtigen Anteile bei 60°C im Wasserstrahlvakuum erhielt man die Titelverbindung in quantitativer Ausbeute mit Zersetzungspunkt 220 - 224°C.

Die in der nachfolgenden Tabelle 1 genannten Wirkstoffe werden auf analogem Herstellungsweg erhalten.

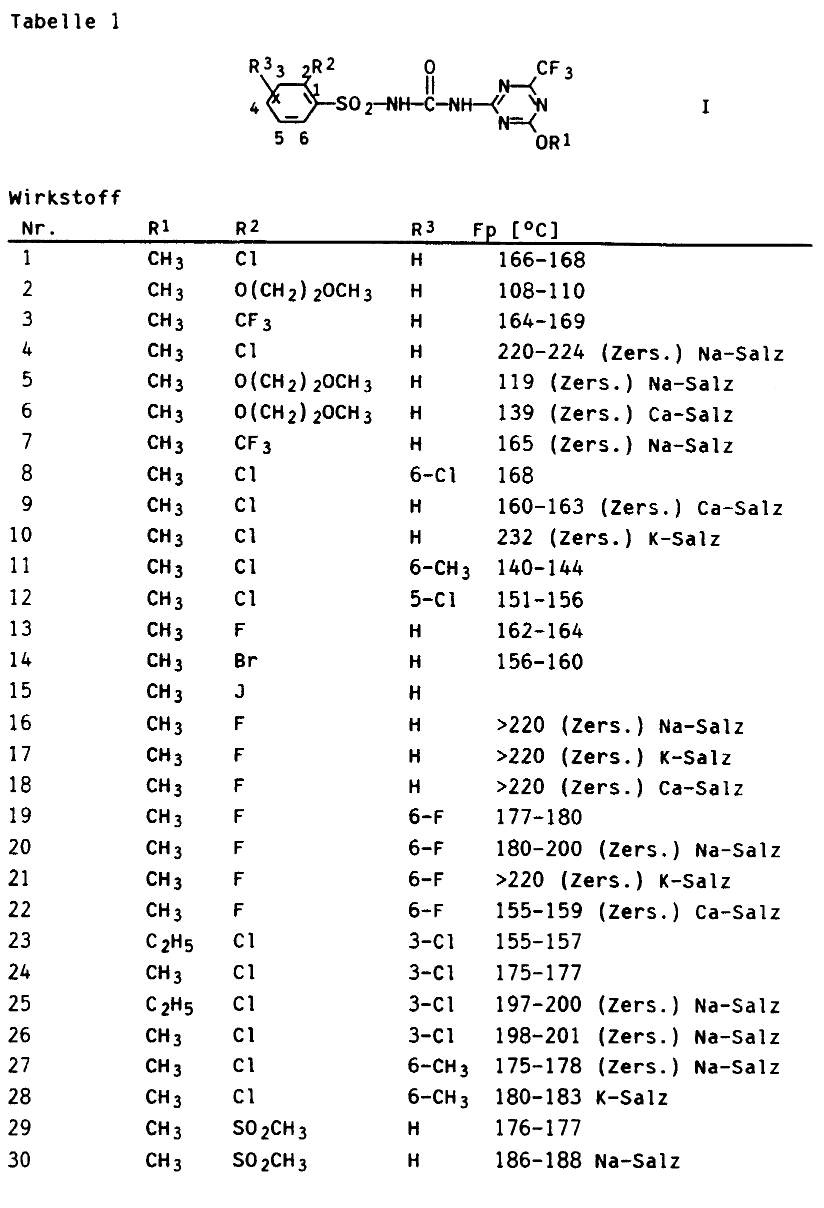

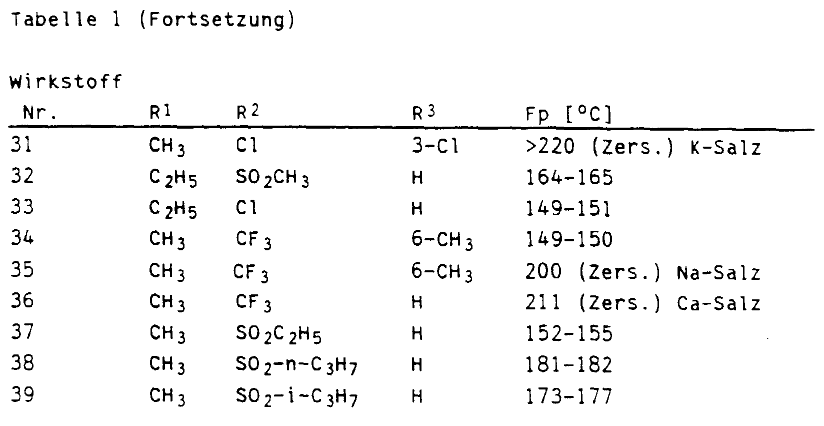

In analoger Weise können auch die nachfolgend aufgeführten Verbindungen erhalten werden: 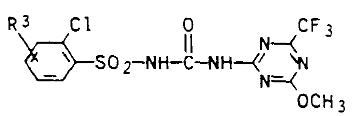 oder deren Na-Salze, wobei R³ die folgende Bedeutung hat:
Wasserstoff, 3-Methyl, 4-Methyl, 5-Methyl, 6-Methyl, 3-Ethyl, 4-Ethyl, 5-Ethyl, 6-Ethyl, 3-Fluor, 4-Fluor, 5-Fluor, 6-Fluor, 3-Chlor, 4-Chlor, 5-Chlor, 6-Chlor, 3-Methoxy, 4-Methoxy, 5-Methoxy, 6-Methoxy, 3-Ethoxy, 4-Ethoxy, 5-Ethoxy, 6-Ethoxy; 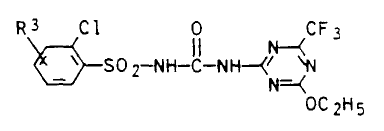 oder deren Na-Salze, wobei R³ die folgende Bedeutung hat:
Wasserstoff, 3-Methyl, 4-Methyl, 5-Methyl, 6-Methyl, 3-Ethyl, 4-Ethyl, 5-Ethyl, 6-Ethyl, 3-Fluor, 4-Fluor, 5-Fluor, 6-Fluor, 3-Chlor, 4-Chlor, 5-Chlor, 6-Chlor, 3-Methoxy, 4-Methoxy, 5-Methoxy, 6-Methoxy, 3-Ethoxy, 4-Ethoxy, 5-Ethoxy, 6-Ethoxy; 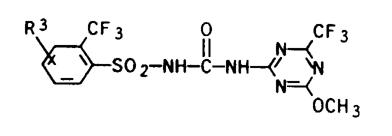 oder deren Na-Salze, wobei R³ die folgende Bedeutung hat:
Wasserstoff, 3-Methyl, 4-Methyl, 5-Methyl, 6-Methyl, 3-Ethyl, 4-Ethyl, 5-Ethyl, 6-Ethyl, 3-Fluor, 4-Fluor, 5-Fluor, 6-Fluor, 3-Chlor, 4-Chlor, 5-Chlor, 6-Chlor, 3-Methoxy, 4-Methoxy, 5-Methoxy, 6-Methoxy, 3-Ethoxy, 4-Ethoxy, 5-Ethoxy, 6-Ethoxy; 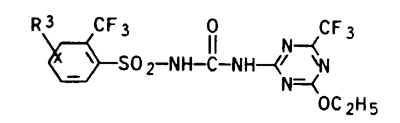 oder deren Na-Salze, wobei R³ die folgende Bedeutung hat:
Wasserstoff, 3-Methyl, 4-Methyl, 5-Methyl, 6-Methyl, 3-Ethyl, 4-Ethyl, 5-Ethyl, 6-Ethyl, 3-Fluor, 4-Fluor, 5-Fluor, 6-Fluor, 3-Chlor, 4-Chlor,
5-Chlor, 6-Chlor, 3-Methoxy, 4-Methoxy, 5-Methoxy, 6-Methoxy, 3-Ethoxy, 4-Ethoxy, 5-Ethoxy, 6-Ethoxy; 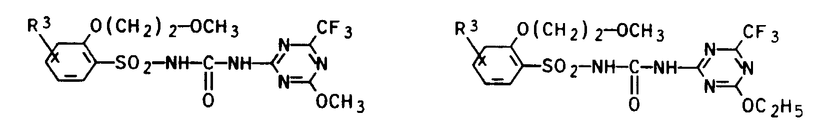 oder deren Na-Salze, wobei R³ die folgende Bedeutung hat:
Wasserstoff, 3-Methyl, 4-Methyl, 5-Methyl, 6-Methyl, 3-Ethyl, 4-Ethyl, 5-Ethyl, 6-Ethyl, 3-Fluor, 4-Fluor, 5-Fluor, 6-Fluor, 3-Chlor, 4-Chlor, 5-Chlor, 6-Chlor, 3-Methoxy, 4-Methoxy, 5-Methoxy, 6-Methoxy, 3-Ethoxy, 4-Ethoxy, 5-Ethoxy, 6-Ethoxy; 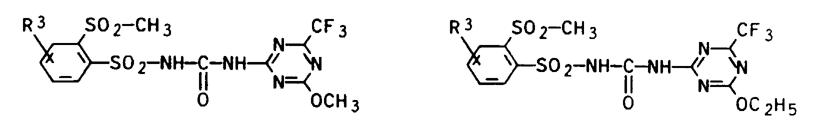 oder deren Na-Salze, wobei R³ die folgende Bedeutung hat:
Wasserstoff, 3-Methyl, 4-Methyl, 5-Methyl, 6-Methyl, 3-Ethyl, 4-Ethyl, 5-Ethyl, 6-Ethyl, 3-Fluor, 4-Fluor, 5-Fluor, 6-Fluor, 3-Chlor, 4-Chlor, 5-Chlor, 6-Chlor, 3-Methoxy, 4-Methoxy, 5-Methoxy, 6-Methoxy, 3-Ethoxy, 4-Ethoxy, 5-Ethoxy, 6-Ethoxy; 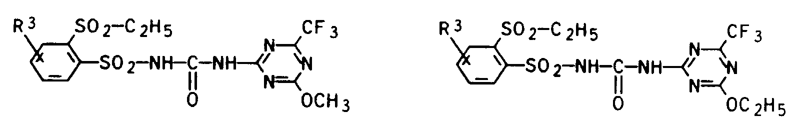 oder deren Na-Salze, wobei R³ die folgende Bedeutung hat:
Wasserstoff, 3-Methyl, 4-Methyl, 5-Methyl, 6-Methyl, 3-Ethyl, 4-Ethyl, 5-Ethyl, 6-Ethyl, 3-Fluor, 4-Fluor, 5-Fluor, 6-Fluor, 3-Chlor, 4-Chlor, 5-Chlor, 6-Chlor, 3-Methoxy, 4-Methoxy, 5-Methoxy, 6-Methoxy, 3-Ethoxy, 4-Ethoxy, 5-Ethoxy, 6-Ethoxy; 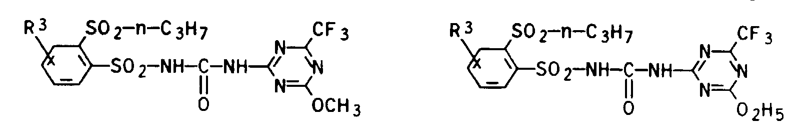 oder deren Na-Salze, wobei R³ die folgende Bedeutung hat:
Wasserstoff, 3-Methyl, 4-Methyl, 5-Methyl, 6-Methyl, 3-Ethyl, 4-Ethyl, 5-Ethyl, 6-Ethyl, 3-Fluor, 4-Fluor, 5-Fluor, 6-Fluor, 3-Chlor, 4-Chlor, 5-Chlor, 6-Chlor, 3-Methoxy, 4-Methoxy, 5-Methoxy, 6-Methoxy, 3-Ethoxy, 4-Ethoxy, 5-Ethoxy, 6-Ethoxy; 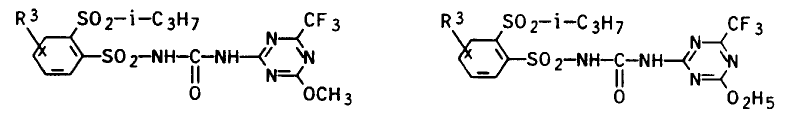 oder deren Na-Salze, wobei R³ die folgende Bedeutung hat:
wasserstoff, 3-Methyl, 4-Methyl, 5-Methyl, 6-Methyl, 3-Ethyl, 4-Ethyl, 5-Ethyl, 6-Ethyl, 3-Fluor, 4-Fluor, 5-Fluor, 6-Fluor, 3-Chlor, 4-Chlor, 5-Chlor, 6-Chlor, 3-Methoxy, 4-Methoxy, 5-Methoxy, 6-Methoxy, 3-Ethoxy, 4-Ethoxy, 5-Ethoxy, 6-Ethoxy.

### Anwendungsbeispiele:

Die herbizide Wirkung der N-[(1,3,5-Triazin-2-yl)aminocarbonyl]-benzolsulfonamide der Formel I auf das Wachstum der Testpflanzen wird durch folgende Gewächshausversuche gezeigt.

Als Kulturgefäße dienen Plastikblumentöpfe mit 300 cm³ Inhalt und lehmigem Sand mit etwa 3,0 % Humus als Substrat. Die Samen der Testpflanzen werden nach Arten getrennt flach eingesät.

Zum Zwecke der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 0,06 bzw. 0,03 kg/ha a.S. (aktive Substanz).

Die Versuchsgefäße werden im Gewächshaus aufgestellt, wobei für wärmeliebende Arten wärmere Bereiche (20 bis 35°C) und für solche gemäßigter Klimate 10 bis 20°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name |
|---|---|
| Amaranthus retroflexus | Zurückgekrümmter Fuchsschwanz |
| Chenopodium album | Weißer Gänsefuß |
| Chrysanthemum | Kronenwucherblume |
| Galium aparine | Klettenlabkraut |
| Stellaria media | Vogelmiere |
| Triticum aestivum | Sommerweizen |
| Zea mays | Mais |

Mit 0,06 bzw. 0,03 kg/ha a.S. im Nachauflaufverfahren eingesetzt lassen sich mit Beispiel Nr. 1 breitblättrige unerwünschte Pflanzen sehr gut bekämpfen, bei gleichzeitiger hervorragender Selektivität in den Kulturpflanzen Weizen und Mais.

In den Tabellen 2 und 3 werden die erfindungsgemäßen Verbindungen der Beispiele 1 bzw. 3 den aus dem US-Patent 4 127 405 bekannten Vergleichssubstanzen G bzw. H gegenübergestellt. 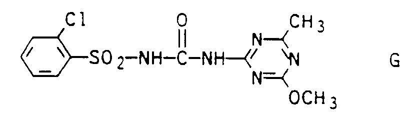 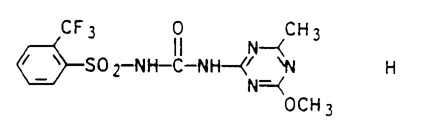 Die Versuchsergebnisse demonstrieren deutlich die überraschend hohen Selektivitäten.

Die bekannten Verbindungen bewirken nicht akzeptable Schädigungen von 85 bzw. 70 % in der Kultur Mais. Demgegenüber zeigen die Beispielverbindungen 1 bzw. 3 bei gleich guter bis besserer herbizider Wirkung nur noch 10 % Schädigung der Kulturpflanze.

**Tabelle 2:**

| Vergleich der herbiziden Aktivität der Beispielverbindung Nr. 1 mit der Vergleichsverbindung G bei Nachauflaufanwendung von 0,03 kg/ha a.S. im Gewächshaus | | |
|---|---|---|
| Testpflanzen | Schädigung [%] | |
| | Beispiel 1 | G |
| Zea mays | 10 | 85 |
| unerwünschte Pflanzen: Amaranthus retroflexus | 90 | 90 |
| Galium aparine | 80 | 74 |

**Tabelle 3:**

| Vergleich der herbiziden Aktivität der Beispielverbindung Nr. 3 mit der Vergleichsverbindung H bei Nachauflaufanwendung von 0,06 bzw. 0,03 kg/ha a.S. im Gewächshaus | | | | |
|---|---|---|---|---|
| Testpflanzen | Schädigung [%] | | | |
| | Aufwandmenge [kg/ha a.S.] | | | |
| | Beispiel 3 | | H | |
| | 0,06 | 0,03 | 0,06 | 0,03 |
| Zea mays | 10 | 10 | 70 | 70 |
| unerwünschte Pflanzen: | | | | |
| Amaranthus retroflexus | 90 | 90 | 90 | 90 |
| Galium aparine | 95 | 60 | 10 | 0 |
| Chenopodium album | 98 | 98 | 98 | 98 |
| Sinapis alba | 95 | 90 | 90 | 90 |

Hervorragende Selektivitäten in den empfindlichen Beispielkulturen Reis, Sommerweizen und Mais werden durch die erfindungsgemäße Verbindung Nr. 7 erzielt, wie die in den nachfolgenden Tabellen 4 und 5 zusammengestellten Ergebnisse zeigen.

**Tabelle 4:**

| Bekämpfung unerwünschter breitblättriger Pflanzen bei gleichzeitiger Verträglichkeit für die Beispielkulturen Sommerweizen und Mais bei Nachauflaufapplikation von 0,015 kg a.S./ha der Verbindung Nr. 7 im Gewächshaus | |
|---|---|
| Testpflanzen | Schädigung [%] |
| Triticum aestivum | 10 |
| Zea mays | 15 |
| unerwünschte Pflanzen: Amaranthus retroflexus | 90 |
| Chenopodium album | 75 |
| Stellaria media | 100 |

**Tabelle 5:**

| Bekämpfung unerwünschter breitblättriger Pflanzen bei gleichzeitiger Verträglichkeit für die Beispielkultur Reis bei Nachauflaufapplikation von 0,015 kg a.S./ha der Verbindung Nr. 7 im Gewächshaus | |
|---|---|
| Testpflanzen | Schädigung [%] |
| Oryza sativa | 10 |
| unerwünschte Pflanzen | |
| Amaranthus retroflexus | 95 |
| Sinapis alba | 70 |
| Stellaria media | 100 |

In einem weiteren Versuch wurde das Natriumsalz der Vergleichsverbindung H dem Beispiel 7 gegenübergestellt. Die Ergebnisse sind in Tabelle 6 zusammengestellt.

**Tabelle 6:**

| Vergleich der herbiziden Aktivität der Beispielverbindung Nr. 7 mit dem Natriumsalz der Vergleichsverbindung H bei Nachauflaufanwendung von 0,06 bzw. 0,03 kg/ha a.S. im Gewächshaus | | | | |
|---|---|---|---|---|
| Testpflanzen | Schädigung [%] | | | |
| | Aufwandmenge [kg/ha a.S.] | | | |
| | Beispiel 7 | | H-Na-Salz | |
| | 0,06 | 0,03 | 0,06 | 0,03 |
| Zea mays | 10 | 0 | 100 | 100 |
| unerwünschte Pflanzen: Amaranthus retroflexus | 100 | 100 | 90 | 90 |
| Galium aparine | 98 | 98 | 70 | 60 |
| Chenopodium album | 100 | 100 | 100 | 100 |

Die Versuchsergebnisse demonstrieren deutlich die überraschend hohe Selektivität bei gleichzeitig hervorragender herbizider Wirksamkeit der erfindungsgemäßen Verbindung.

## Patentansprüche

1. N-[(1, 3, 5-Triazin-2-yl)aminocarbonyl]benzolsulfonamide der allgemeinen Formel I 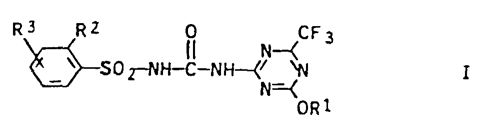 in der R¹ eine Methyl- oder Ethylgruppe, R² Halogen, eine C₁-C₃-Alkylsulfonylgruppe, die Trifluormethylgruppe oder 2-Methoxyethoxygruppe und R³ Wasserstoff, die Methyl-, Ethyl-, Methoxy- oder Ethoxygruppe, Fluor oder Chlor bedeuten, sowie deren landwirtschaftlich brauchbare Salze.

2. N-[(1,3,5-Triazin-2-yl)aminocarbonyl]benzolsulfonamide der Formel I gemäß Anspruch 1, in der R² Chlor bedeutet.

3. Verfahren zur Herstellung der N-[(1,3,5-Triazin-2-yl)amino-carbonyl]benzolsulfonamide der Formel I gemäß Anspruch 1, dadurch gekennzeichnet, daß man ein Sulfonylisocyanat II 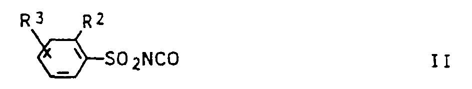 in an sich bekannter Weise in einem inerten organischen Lösungsmittel mit der ungefähr stöchiometrischen Menge eines 2-Amino-1,3,5-triazin-derivats III 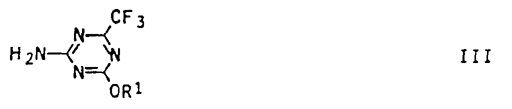 umsetzt.

4. Verfahren zur Herstellung der N-[(1,3,5-Triazin-2-yl)amino-carbonyl]benzolsulfonamide der Formel I gemäß Anspruch 1, dadurch gekennzeichnet, daß man ein Carbamat der Formel IV 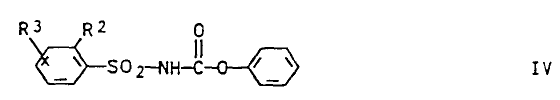 in an sich bekannter Weise in einem inerten organischen Lösungsmittel bei einer Temperatur zwischen 0 und 120°C mit ungefähr der stöchiometrischen Menge eines 2-Amino-1,3,5-triazins III 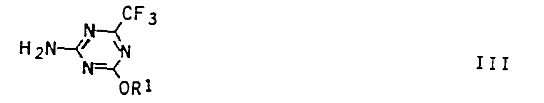 umsetzt.

5. Verfahren zur Herstellung der N-[(1,3,5-Triazin-2-yl)amino-carbonyl]benzolsulfonamide der Formel I gemäß Anspruch 1, dadurch gekennzeichnet, daß man ein entsprechendes Sulfonamid der Formel V 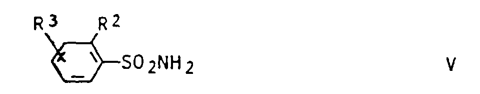 in an sich bekannter Weise in einem inerten organischen Lösungsmittel mit einem Phenylcarbamat VI 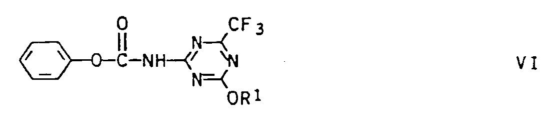 umsetzt.

6. Verfahren zur Herstellung der N-[(1,3,5-Triazin-2-yl)amino-carbonyl]benzolsulfonamide der Formel I gemäß Anspruch 1, dadurch gekennzeichnet, daß man ein entsprechendes Sulfonamid der Formel V 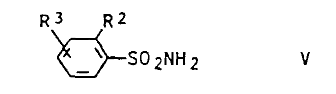 in an sich bekannter Weise in einem inerten organischen Lösungsmittel mit einem Isocyanat der Formel VII 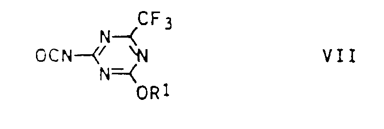 umsetzt.

7. Verfahren zur Herstellung von Salzen der Verbindungen I gemäß Anspruch 1, dadurch gekennzeichnet, daß man eine Verbindung der Formel I gemäß Anspruch 1 in an sich bekannter Weise in Wasser oder einem inerten organischen Lösungsmittel mit einer Base deprotoniert.

8. Herbizides Mittel, enthaltend ein N-[(1,3,5-Triazin-2-yl)-aminocarbonyl]benzolsulfonamid der Formel I gemäß Anspruch 1 oder sein Salz sowie hierfür übliche Trägerstoffe.

9. Verfahren zur Bekämpfung unerwünschten Pflanzenwuchses, dadurch gekennzeichnet, daß man eine herbizid wirksame Menge eines N-[(1,3,5-Triazin-2-yl)aminocarbonyl]benzolsulfonamids der Formel I gemäß Anspruch 1 oder eines seiner Salze auf die Pflanzen und/oder ihren Lebensraum einwirken läßt.

10. Verwendung der Verbindungen I gemäß Anspruch 1 als herbizide Mittel.

## Claims

1. An N-[(1,3,5-triazin-2-yl)-aminocarbonyl]-benzenesulfonamide of the formula I 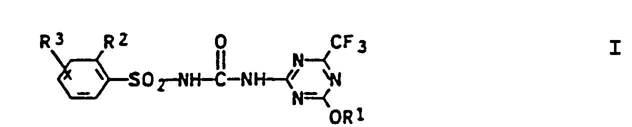 where R¹ is methyl or ethyl, R² is halogen, C₁-C₃-alkyl-sulfonyl, trifluoromethyl or 2-methoxyethoxy and R³ is hydrogen, methyl, ethyl, methoxy, ethoxy, fluorine or chlorine, or its agriculturally useful salts.

2. An N-[(1,3,5-triazin-2-yl)-aminocarbonyl]-benzenesulfonamide of the formula I as claimed in claim 1, where R² is chlorine.

3. A process for the preparation of an N-[(1,3,5-triazin-2-yl)-aminocarbonyl]-benzenesulfonamide of the formula I as claimed in claim 1, wherein a sulfonyl isocyanate II 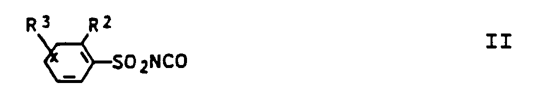 is reacted in a conventional manner in an inert organic solvent with about the stoichiometric amount of a 2-amino-1,3,5-triazine derivative III

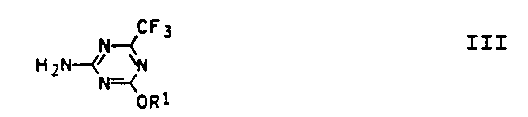

4. A process for the preparation of an N-[(1,3,5-triazin-2-yl)-aminocarbonyl]-benzenesulfonamide of the formula I as claimed in claim 1, wherein a carbamate of the formula IV 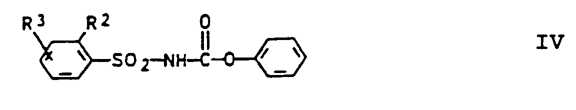 is reacted in a conventional manner in an inert organic solvent at from 0 to 120°C with about the stoichiometric amount of a 2-amino-1,3,5-triazin III

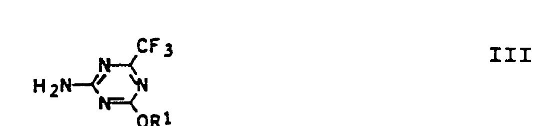

5. A process for the preparation of an N-[(1,3,5-triazin-2-yl)-aminocarbonyl]-benzenesulfonamide of the formula I as claimed in claim 1, wherein a corresponding sulfonamide of the formula V 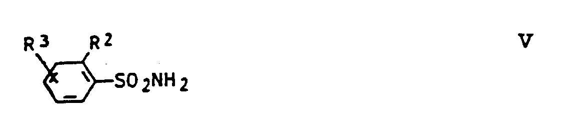 is reacted in a conventional manner in an inert organic solvent with a phenyl carbamate VI

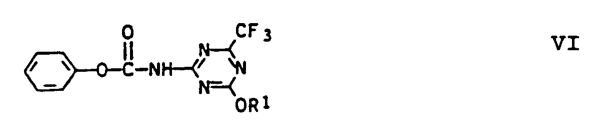

6. A process for the preparation of an N-[(1,3,5-triazin-2-yl)-aminocarbonyl]-benzenesulfonamide of the formula I as claimed in claim 1, wherein a corresponding sulfonamide of the formula V 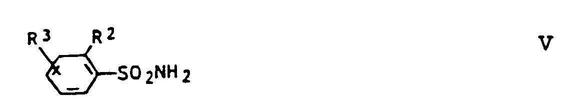 is reacted in a conventional manner in an inert organic solvent with an isocyanate of the formula VII

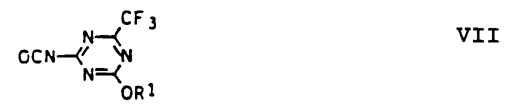

7. A process for the preparation of a salt of a compound I as claimed in claim 1, wherein a compound of the formula I as claimed claim 1 is deprotonated in a conventional manner in water or in an inert organic solvent with a base.

8. A herbicide containing an N-[(1,3,5-triazin-2-yl) -aminocarbonyl] -benzenesulfonamide of the formula I as claimed in claim 1 or its salt and conventional carriers.

9. A method for controlling undesirable plant growth, wherein a herbicidal amount of an N-[(1,3,5-triazin-2-yl)-aminocarbonyl]-benzenesulfonamide of the formula I as claimed in claim 1 or of one of its salts is allowed to act on the plants and/or their habitat.

10. Use of a compound I as claimed in claim 1 as a herbicide.

## Revendications

1. N-/(1,3,5-triazin-2-yl)aminocarbonyl/benzènesulfonamides de formule générale 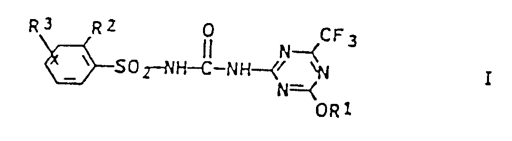 dans laquelle R¹ représente un groupe méthyle ou éthyle, R² un halogène, un groupe alkylsulfonyle en C1-C3, le groupe trifluorométhyle ou le groupe 2-méthoxyéthoxy et R³ représente l'hydrogène, un groupe méthyle, éthyle, méthoxy ou éthoxy, le fluor ou le chlore, et leurs sels convenant pour les applications agricoles.

2. N-/(1,3,5-triazin-2-yl)aminocarbonyl/benzénesulfonamides de formule I selon revendication 1 dans laquelle R² représente le chlore.

3. Procédé de préparation des N-/(1,3,5-triazin-2-yl)aminocarbonyl/benzènesulfonamides de formule I de la revendication 1, caractérisé par le fait que l'on fait réagir un sulfonylisocyanate II 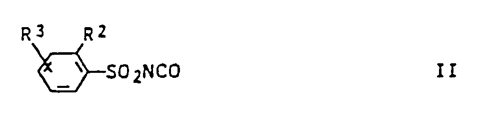 de manière connue en soi, dans un solvant organique inerte, avec une quantité à peu près stoechiométrique d'un dérivé de la 2-amino-1,3,5-triazine III

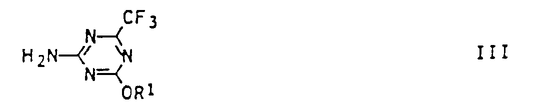

4. Procédé de préparation des N-/(1,3,5-triazin-2-yl) aminocarbonyl/benzènesulfonamides de formule I de la revendication 1, caractérisé par le fait que l'on fait réagir un carbamate de formule IV 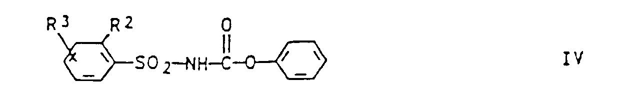 de manière connue en soi, dans un solvant organique inerte, à une température allant de 0 à 120 ° C, avec la quantité à peu près stoechiométrique d'une 2-amino-1,3,5-triazine III

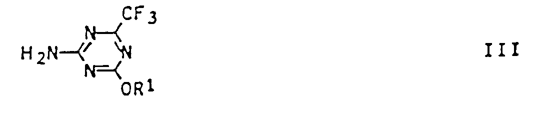

5. Procédé de préparation des N-/(1,3,5-triazin-2-yl)aminocarbonyl/benzènesulfonamides de formule I de la revendication 1, caractérisé par le fait que l'on fait réagir un sulfonamide correspondant de formule V 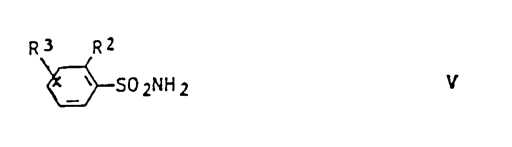 de manière connue en soi, dans un solvant inerte, avec un phénylcarbamate VI

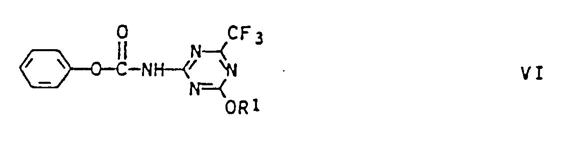

6. Procédé de préparation des N-/(1,3,5-triazin-2-yl)aminocarbonyl /benzènesulfonamides de formule I de la revendication 1, caractérisé par le fait que l'on fait réagir un sullfonamide correspondant de formule V 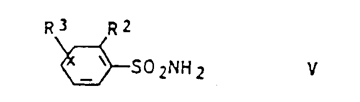 de manière connue en soi, dans un solvant organique inerte, avec un isocyanate de formule VII

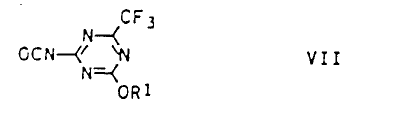

7. Procédé de préparation des sels des composés I de la revendication 1, caractérisé par le fait que l'on déprotonise un composé de formule I de la revendication 1, de manière connue en soi, dans l'eau ou dans un solvant organique inerte, à l'aide d'une base.

8. Produit herbicide contenant un N-/( 1,3,5-triazin-2-yl)aminocarbonyl/benzènesulfonamide de formule I de la revendication 1 ou l'un de ses sels et des véhicules usuels à cet effet.

9. Procédé pour combattre les croissances de végétaux indésirables, caractérisé par le fait que l'on fait agir une quantité herbicide d'un N-/(1,3,5-triazin-2-yl)-aminocarbonyl/benzènesulfonamide de formule I de la revendication 1 ou de l'un de ses sels sur les végétaux et/ou leur habitat.

10. Utilisation des composés I de la revendication 1 en tant que produits herbicides.
